# DEMANDE DE BREVET EUROPEEN

(11) **EP 3 581 210 A2**
(43) Date de publication de la demande: **18.12.2019**
(21) Numéro de dépôt: 19168089.1
(22) Date de dépôt: 16.09.2015
(51) Int. Cl.: A61L 9/12, B60H 3/00

(54) **CARTOUCHE AMOVIBLE POUR DISPOSITIF DE DIFFUSION**

(30) Priorité: 30.09.2014 FR 1459330
(62) Demande divisionnaire de: 15770485.9
(71) Demandeur: Valeo Systemes Thermiques, 78320 Le Mesnil Saint Denis (FR)
(72) Inventeur: QUEINNEC, Jean-Yves, 78322 LE MESNIL SAINT-DENIS CEDEX (FR); LECHAT, Yvan, 78322 LE MESNIL SAINT-DENIS CEDEX (FR)
(74) Mandataire: Metz, Gaëlle

(57) **Abrégé**

L'invention concerne une cartouche (130) comprenant un ou une pluralité d'agents volatils, notamment de fragrances, ledit agent ou chacun desdits agents étant logés dans une cavité (340) destinée à être balayée par un flux d'air (120), ladite cartouche étant configurée pour équiper un dispositif de diffusion, en particulier pour véhicules automobiles.

## Description

La présente invention concerne un dispositif de diffusion, en particulier pour véhicules automobiles, et une cartouche amovible pour un tel dispositif.

Une amélioration de la qualité de l'air d'un véhicule automobile est d'une importance primordiale pour un passager. Une meilleure qualité d'air implique non seulement une amélioration du confort du passager mais peut également avoir des répercussions sur la santé du passager. Différents dispositif de diffusion permettant un traitement de l'air de la cabine de véhicule existent. Ces derniers peuvent être directement reliés au système de ventilation, de chauffage ou de climatisation mais ils peuvent également être des dispositifs indépendants des systèmes préexistants tels que ceux cités ci-dessus. Dans ce cas, ils sont souvent dissimulés sous la planche de bord, par exemple dans une boîte à gants ou vide-poche. L'air traité, ou parfumé, ressort par une grille de ventilation pouvant être fixée sur la planche de bord ou ailleurs.

De tels dispositifs sont pourvus d'un système permettant d'y insérer des cartouches comprenant un agent volatil tout en laissant un flux d'air entrer en contact ou non avec ledit agent. Cependant l'orientation du flux d'air qui les traverse ne permet pas d'optimiser l'exploitation de l'agent volatil.

L'invention se propose d'améliorer les inconvénients présentés ci-dessus grâce à un dispositif de diffusion, en particulier pour véhicules automobiles, ledit dispositif étant configuré pour accueillir une cartouche comprenant un ou une pluralité d'agents volatils, notamment de fragrances, ledit agent ou desdits agents étant logé dans une cavité destinée à être balayée par un flux d'air, ledit dispositif comprenant en outre un sélecteur muni d'une entrée et d'une sortie dudit flux d'air, ledit sélecteur configuré pour être mobile par rapport à ladite cartouche de manière à mettre en communication ladite entrée et ladite sortie, avec ladite ou l'une au moins desdites cavités pour que cette dernière soit traversée par ledit flux d'air.

Grâce à l'invention, le flux d'air traverse la ou les cavités chargées d'agents volatils. Le flux d'air est ainsi mis en contact avec le ou les agents volatils de façon plus efficace que s'il ne faisait que lécher l'une des faces de la cartouche.

Bien naturellement, l'agent volatil, notamment la fragrance, contenu dans la ou chacune des cavités pourra comporter un seul ou un mélange de composés chimiques, naturels et/ou artificiels, destinés à traiter, notamment à parfumer, le flux d'air.

Selon différents modes de réalisation de l'invention qui pourront être pris ensemble ou séparément :
- le dispositif comprend en outre un pulseur destiné à générer un flux d'air,
- le dispositif comprend en outre un boîtier dans lequel le sélecteur et/ou le pulseur sont situés,
- le boîtier est configuré pour accueillir ladite cartouche, de préférence de façon amovible,
- le sélecteur comprend deux plateaux tournants disposés de part et d'autre de la cartouche, un premier desdits plateaux tournant comprenant ladite entrée du flux d'air et un second desdits plateaux comprenant ladite sortie du flux d'air,
- l'entraînement desdits plateaux est effectué par un axe mobile unique,
- ladite entrée et/ou ladite sortie du flux d'air sont définies par une ouverture dont la taille est comparable, voire identique, entre elles et/ou à celle d'une ouverture d'entrée et/ou de sortie dudit flux d'air des cavités de la cartouche,
- les ouvertures des plateaux tournants sont situées en vis-à-vis l'une de l'autre,
- le dispositif comprend en outre un mécanisme d'actionnement du sélecteur,
- le mécanisme d'actionnement comprend un moteur et un organe de démultiplication placé entre ledit moteur et ledit sélecteur,
- l'axe d'entraînement est relié audit démultiplicateur,
- ledit dispositif comprend un système de commande, apte à être contrôlé par un utilisateur, ledit système de commande étant relié au mécanisme d'actionnement,
- le dispositif comprend en outre des ressorts pressant les plateaux contre la cartouche.

L'invention concerne aussi une cartouche configurée pour équiper un dispositif tel que décrit plus haut.

Dans le cas d'une cartouche accueillant plusieurs agents volatils, il sera possible de changer plus facilement d'agents volatils, en particulier de fragrances, selon les envies, ou selon les goûts des différents conducteurs ou des différents passagers du véhicule.

Selon différentes caractéristiques supplémentaires de l'invention qui pourront être prises ensemble ou séparément :
- ladite cartouche est configurée pour être amovible par rapport audit dispositif,
- la cartouche comprend une façade d'introduction dans ledit boîtier,
- ladite cartouche comprend au moins trois cavités,
- au moins une desdites cavités de ladite cartouche est vide,
- les différentes cavités de la cartouche forment des portions angulaires d'un anneau,
- la cartouche comprend une fente permettant le passage de la cartouche en présence du sélecteur, en particulier de son axe d'entraînement, lorsque cette dernière est insérée dans le boîtier,
- ladite cartouche comprend un corps et un couvercle,
- ledit corps est compartimenté pour définir lesdites cavités,
- un fond dudit corps comprend les entrées du flux d'air desdites cavités,
- le couvercle comprend les sorties du flux d'air desdites cavités,
- les agents volatils comprennent des billes odorantes.

L'invention concerne encore l'ensemble d'un dispositif et d'une cartouche tels que décrits plus haut.

L'invention sera mieux comprise à la lumière de la description suivante qui n'est donnée qu'à titre indicatif et qui n'a pas pour but de la limiter, accompagnée des dessins joints parmi lesquels :
- la figure 1 illustre une vue globale en perspective d'un dispositif de diffusion selon l'invention,
- la figure 2 illustre le dispositif de la figure 1 sans son boîtier,
- la figure 3 illustre une vue en perspective de la cartouche du dispositif de la figure 1,
- la figure 4 illustre de façon schématique, en vue de dessus, les différentes positions du sélecteur du dispositif de la figure 1.

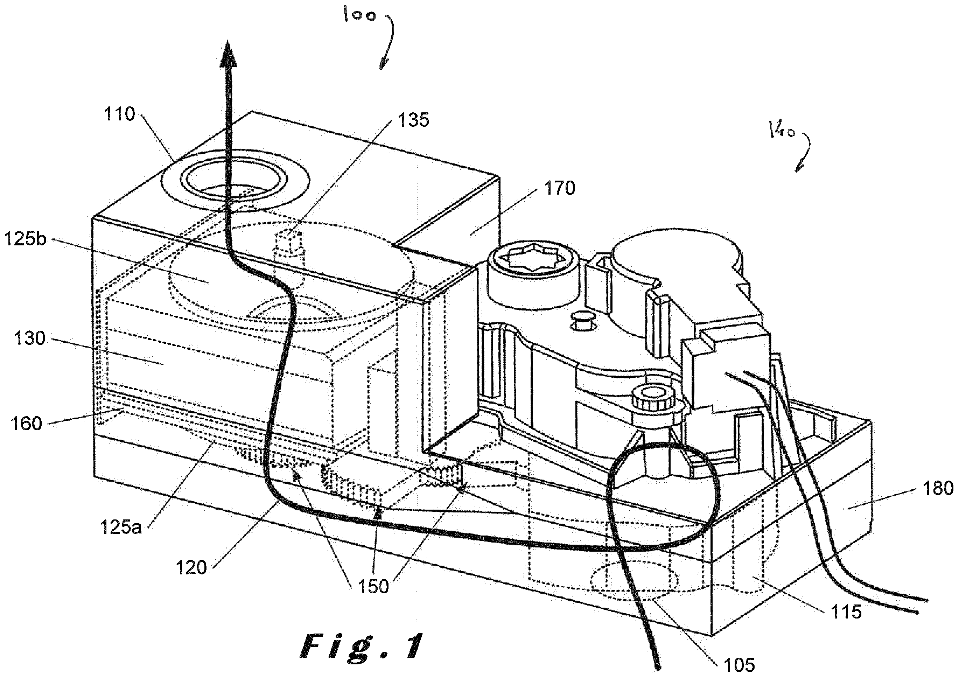

Comme illustré sur la figure 1, l'invention concerne un dispositif de diffusion ou diffuseur permettant le traitement d'un flux d'air à l'aide d'agents volatils, notamment de fragrances.

Selon un mode de réalisation particulièrement avantageux de l'invention, les agents volatils, notamment les fragrances, comprennent un substrat, notamment des billes, chargé en composé(s) volatil(s). Il pourra en particulier s'agir de billes odorantes.

Le diffuseur est illustré sur cette figure dans son ensemble. Il comprend ici un boîtier 100 sur lequel deux orifices servent d'entrée 105 et de sortie 110 de l'air. Dès lors, le boîtier sert de guide d'air et comprend une pluralité de composants qui vont permettre à l'air de circuler dans le boîtier. De préférence, une interface sur les orifices 105, 110 permet à une tubulure, non représentée, de s'y fixer tout en assurant l'étanchéité du système à l'air.

Le diffuseur conforme à l'invention est configuré pour accueillir une cartouche 130 de fragrances et il comprend un sélecteur, ici logé dans ledit boîtier 100. Le sélecteur comprend, par exemple, un sélecteur d'entrée 125a, un sélecteur de sortie 125b et un axe mobile 135 les entraînant. Ledit diffuseur comprend en outre ici un mécanisme d'actionnement 150, permettant un entraînement dudit axe 135, le sélecteur étant prévu mobile par rapport à ladite cartouche.

Le boîtier 100 peut être fixé à différents emplacements du véhicule, de préférence sur la planche de bord ou dans la boîte à gants afin que ce dernier soit facilement accessible au passager. En effet, selon un aspect de l'invention, la cartouche est amovible par rapport audit boîtier et il est avantageux que le passager puisse facilement changer la cartouche.

La sortie d'air du boîtier 110 est reliée, par exemple, à une grille par laquelle l'air traité ou parfumé sort. Cette grille peut être fixée à différents emplacements dans le véhicule. De préférence, elle est située sur la planche de bord ou près des systèmes de ventilation du pare-brise, la grille étant reliée à la sortie du boîtier par la tubulure déjà évoquée.

Ici, le boîtier 100 comprend une base 180, par exemple parallélépipède rectangle. Dans la base sont logés un pulseur 115 situé au-dessus de l'entrée 105 du boîtier et au moins une partie du mécanisme d'actionnement 150. Ledit mécanisme d'actionnement pourra comprendre un moteur 140. Ce dernier est fixé sur le boîtier 100 au-dessus du pulseur 115, et se trouve en dehors du boîtier. Au-dessus de la base 180, et à côté du moteur 140, une seconde partie du boîtier 170 possède une forme sensiblement cubique pour y loger le sélecteur de fragrances et la cartouche 130. Ces parties du boîtier sont assemblées pour n'en former qu'une seule. L'étanchéité à l'air est assurée dans l'ensemble du boîtier. L'orifice d'entrée 105 est donc dans la base du boîtier 180, sur la face inférieure et en dessous du pulseur 115. L'orifice de sortie 110 est sur la face supérieure de la deuxième partie 170, au-dessus du sélecteur de sortie 125b.

L'air pénètre dans le boîtier par l'orifice d'entrée 105, en étant aspiré par le pulseur 115. Ce pulseur génère le flux d'air qui va finalement sortir du boîtier par l'orifice de sortie 110 après avoir traversé la cartouche 130. Le flux d'air est illustré par la ligne 120 sur la figure 1. De préférence, le pulseur 115 est de type radial, mais il peut également être de type axial.

Selon l'invention, la cartouche 130 est composée d'un ou d'une pluralité de cavités dans lesquelles différentes fragrances peuvent être logées. Le sélecteur 125a, 125b est configuré de sorte que l'une ou plusieurs de ces cavités soient traversées par le flux d'air afin que celui-ci soit chargé des composés volatils de la ou des fragrances sélectionnées. Autrement dit, lorsque le flux d'air traverse la ou lesdites cavités sélectionnées, l'air vient en contact avec l'ensemble du substrat contenu dans chacune desdites cavités et ledit substrat libère le ou les composés qui vont parfumer et/ou traiter le flux d'air, et ensuite l'habitacle du véhicule automobile. Le sélecteur sert par ailleurs à fermer la ou les autres cavités. Plus précisément, dans le cas d'une cartouche munie d'une seule cavité présentant un agent volatil, il pourra servir à fermer ladite cavité, dans une autre position de ses entrée/sortie du flux d'air. Dans le cas d'une cartouche munie de plusieurs cavités présentant un agent volatil, il servira à fermer les autres cavités.

Pour cela, le flux d'air 120 pénètre dans la cartouche 130 par une entrée du sélecteur d'entrée. Le flux d'air traverse alors la cartouche 130 de part en part en passant par l'une ou plusieurs des cavités de ladite cartouche qu'il quitte à travers une sortie dudit sélecteur de sortie 125b. En faisant traverser la ou les cavités sélectionnées par le flux d'air, on s'assure d'un chargement optimisé du flux d'air par la fragrance, celui-ci venant au contact de l'ensemble de la surface du substrat associé. Après être sorti de la cartouche, le flux d'air est ici guidé jusqu'à l'orifice de sortie 110 du boîtier.

De manière préférentielle, le pulseur 115 permet au dispositif d'être indépendant du système de ventilation, de climatisation ou de chauffage dont le véhicule est éventuellement équipé. Il peut dès lors être utilisé indépendamment de ces systèmes, ce qui le rend d'autant plus flexible à l'utilisation. Dans un autre mode de réalisation de la présente invention, il est relié au système de ventilation, de climatisation ou de chauffage du véhicule. L'orifice d'entrée 105 et de sortie 110 seraient dans ce mode de réalisation reliés à un conduit dudit système.

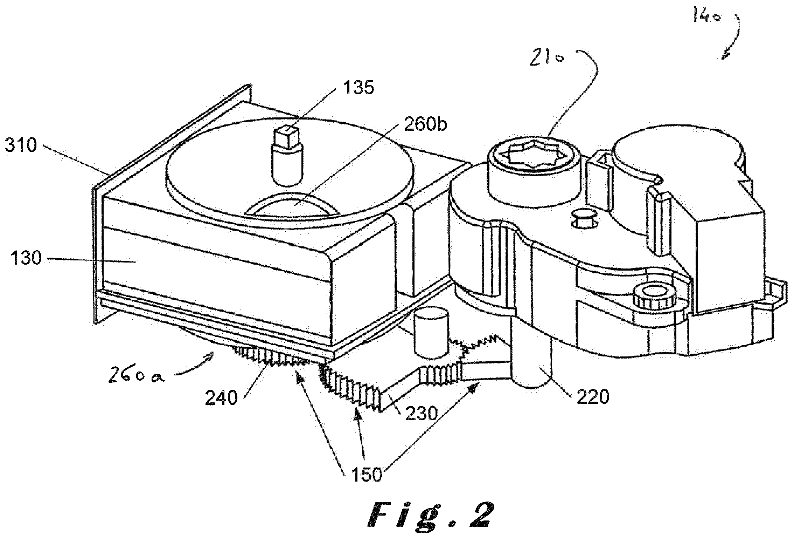

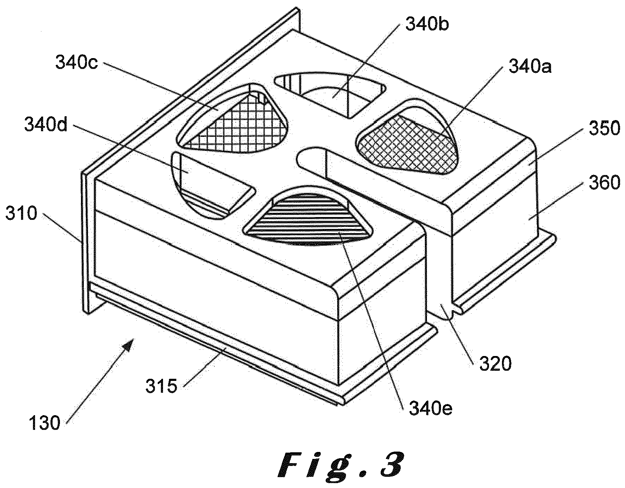

La figure 2 offre une vue plus détaillée de la figure 1 sans le boîtier 100. La figure 2 illustre les détails du mécanisme d'actionnement 150, de l'axe mobile 135 et du sélecteur d'entrée 125a et de sortie 125b. La cartouche 130 est illustrée plus en détails sur la figure 3.

Le mécanisme d'actionnement 150 comprend par exemple le moteur 140, un arbre 220, un pignon de transmission 230, permettent avantageusement une démultiplication, et un pignon droit 240. Le moteur 140 entraîne la cinématique des éléments situés en aval : l'arbre 220, directement relié à un actuateur du moteur, entraîne le pignon de transmission 230, qui entraîne le pignon droit 240. L'axe mobile 135 est directement fixé sur le pignon droit 240 et est dès lors également entraîné par ce dernier.

Avantageusement, le sélecteur d'entrée 125a et le sélecteur de sortie 125b se présentent sous la forme de plateaux tournants, l'axe mobile 135 perçant les deux plateaux tournants en leur centre. La forme du trou perçant les plateaux tournants correspond à la forme d'une section de l'axe mobile, de préférence de forme carrée de telle sorte que lorsque l'axe mobile tourne, les plateaux tournants tournent également. Les plateaux tournants sont séparés par une distance correspondant à la hauteur de la cartouche 130 afin de permettre à la cartouche d'y être insérée. Lorsque la cartouche est insérée entre les deux plateaux, ces derniers sont avantageusement pressés contre la cartouche par des ressorts, non illustrés, afin d'assurer l'étanchéité à l'air.

De préférence, l'entrée et la sortie du flux d'air sur les plateaux tournants situés respectivement en amont et en aval de la cartouche se présentent sous la forme d'ouvertures débouchantes 260b (l'ouverture 260a en amont de la cartouche n'est pas visible) dont la taille, avantageusement prévue identique, est comparable à celle d'une entrée, respectivement d'une sortie d'air, d'une cavité de la cartouche. Les ouvertures des deux plateaux tournants sont disposées en vis-à-vis de manière à assurer la continuité du flux d'air. D'autre part, les positions des ouvertures desdits plateaux tournants restent synchronisées pendant leur mouvement grâce à l'axe mobile 135 qui les relie.

La figure 3 offre une vue de la cartouche isolée 130. Avantageusement, la cartouche 130 est un élément amovible par rapport au boîtier 100. Le passager du véhicule peut alors facilement changer de cartouche, notamment lorsqu'elle est vide, la durée de vie d'une cartouche étant d'environ 3 mois pour une utilisation normale. Par « vide », on entend dont les fragrances sont épuisées et ne libèrent plus de composés volatils.

La cartouche est ici munie d'une façade d'introduction 310 dans ledit boîtier, se présentant de manière similaire à la façade d'un tiroir. Cette façade 310 facilite l'insertion de ladite cartouche 130 dans le boîtier 100. La cartouche 130 est également pourvue d'une nervure longitudinale 315 de guidage située le long des trois faces verticales qui vont s'insérer dans le boîtier 100. Pour accueillir lesdites nervures de guidage 315 de la cartouche 130, le boîtier possède également des rainures de guidage 160, illustrées sur la figure 1, de part et d'autre du logement de la cartouche dans le boîtier et au-dessus du plateau tournant inférieur 125a.

Une fente 320 permet le passage de l'axe lorsque la cartouche est insérée dans le boîtier entre les deux plateaux tournants 125a et 125b illustrés aux figures 1 et 2. En position d'utilisation, l'axe mobile 135 se trouve au centre de la cartouche dans le fond de la fente.

La cartouche comprend un corps 360 et un couvercle 350 dont la forme est parallélépipédique et la base est sensiblement carrée. Le corps est compartimenté pour définir lesdites cavités, le fond dudit corps comprend les entrées du flux d'air desdites cavités, et le couvercle comprend les sorties du flux d'air desdites cavités. La cartouche est de préférence en matière plastique. Lesdites ouvertures pourront être revêtues d'un grillage ou d'un film poreux pour retenir le substrat desdites fragrances tout en laissant passer le flux d'air à travers.

Avantageusement, des cavités occupées par une fragrance et des cavités vides alternent. L'alternance de cavités vides et de cavités logeant une fragrance permet de passer de chacune des cavités comprenant un fragrance à une cavité n'en comportant pas, sans passer par une cavité comprenant une autre fragrance.

Dans un mode de réalisation de la présente invention tel qu'illustré sur la figure 3, les différentes cavités de la cartouche et leurs ouvertures respectives forment des portions angulaires d'un anneau. Dans ce mode de réalisation particulièrement avantageux, cinq cavités sont prévues, dont trois pour loger une fragrance 340a, 340c, 340e et deux sont laissées vides intentionnellement 340b, 340d. L'angle balayé par les ouvertures de chaque portion angulaire est dans ce mode de réalisation légèrement inférieur à 60 degrés. Dans un tel mode de réalisation, les ouvertures 260a, 260b des plateaux se présentent également sous la forme d'une portion angulaire, chaque plateau comprenant ici une unique ouverture 260a, respectivement 260b.

Le passager peut dès lors choisir entre trois fragrances différentes selon ses goûts : différentes ambiances sont envisageables pendant le voyage, ce qui peut être particulièrement agréable pour les longs voyages. Ces différentes fragrances peuvent également être très utiles pour masquer ou filtrer une odeur indésirable de tabac par exemple. Le passager peut également choisir de laisser le système de ventilation en marche sans pour autant être en présence d'une fragrance.

Une telle distribution des cavités, sous la forme de portions angulaires en anneau, permet de facilement augmenter le nombre de cavités sans pour autant devoir modifier le mécanisme d'actionnement associé. Le seul changement à effectuer pour augmenter le nombre de fragrances disponibles au passager réside dans la taille des cavités de la cartouche et dans la taille des ouvertures des plateaux tournants qui leur correspondent.

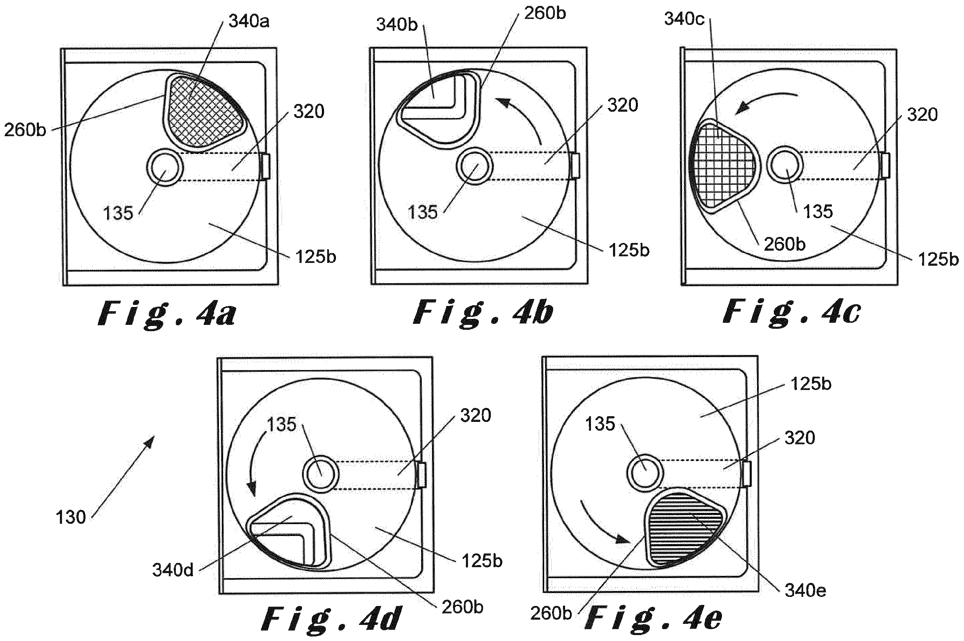

Les figures 4a à 4b illustrent une vue du haut du mécanisme permettant le changement de fragrances effectué par l'ensemble comprenant la cartouche 130 et le sélecteur 125a, 125b. La figure ne montre que le plateau tournant 125b situé sur la cartouche 130. Cependant, la vue du bas serait représentée de la même manière. L'axe mobile 135 ainsi que la fente 320 de la cartouche 130 sont également visibles sur la figure.

Dans le mode de réalisation de la présente invention décrit à la figure 3, où la cartouche comprend cinq cavités dont trois sont dédiées à loger une fragrance et deux sont laissées vides, les figures 4a à 4b illustrent le passage de l'ouverture 260b de la plaque tournante 125b sur chacune des cinq positions. La figure 4a illustre la première position de la plaque tournante où la fragrance libérée provient de la cavité 340a de la figure 3. Pour passer à la deuxième position, illustrée sur la figure 4b, la plaque tournante va tourner dans le sens antihoraire d'un angle d'environ 60 degrés. Cette position correspond à une cavité vide de la cartouche correspondant à la cavité 340b de la figure 3. La figure 4c illustre l'effet d'une rotation supplémentaire dans le sens antihoraire d'environ 60 degrés par rapport à la deuxième position permettant de libérer la deuxième fragrance logée dans la cavité 340c. De la même manière, les figures 4d et 4e illustrent l'ouverture du plateau tournant située respectivement sur les cavités 340d pour le quatrième position et 340e pour la cinquième position de la cartouche.

Préférentiellement, pour passer à une position ultérieure, la plaque tournante va tourner dans le sens horaire. En effet, il y a une zone par laquelle la plaque tournante ne passe pas pendant son utilisation. Cette zone se trouve au-dessus de la fente 320, ou entre les cavités 340a et 340e.

L'alternance de cavités vides et de cavités logeant une fragrance permet au passager de régler l'intensité de la fragrance diffusée par un actionnement du sélecteur en va-et-vient. Dans le même but, il est également possible de placer l'ouverture du plateau tournant en une position intermédiaire entre la cavité vide et la cavité remplie. Lorsque l'ouverture recouvre en totalité l'ouverture de la cavité, alors la diffusion est totale, lorsque l'ouverture recouvre seulement une partie de l'ouverture de la cavité comportant l'agent volatil, alors la diffusion n'est que partielle.

Dans une mode de réalisation préféré de l'invention, la vitesse du plateau tournant pour passer d'une fragrance à une utilisation sans fragrance est de 0.5 seconde. Par suite, la vitesse de transition pour passer de la première position à la cinquième position, et vice-versa, est de 2.5 secondes.

Ce changement rapide entre différentes positions est rendu possible grâce au mécanisme d'actionnement 140 illustré sur les figures 1 et 2, et déjà décrit en partie.

Dans un mode de réalisation préféré, le moteur 140 est de type moto-réducteur pas à pas. Ce type de moteur standard est choisi pour des raisons de coût et de disponibilité sur le marché. Comme illustré sur les figures 1 et 2, l'arbre et le pignon de transmission sont à portion angulaire. En effet, la plage de rotation des plateaux tournants n'est pas de 360°, mais de 240° comme discuté ci-dessus.

C'est la combinaison de l'arbre 220, du pignon de transmission 230 et du pignon droit 240 qui permet une démultiplication du mouvement induit par le moto-réducteur 210 de telle sorte que le sélecteur passe d'une position à la suivante en 0.5 secondes et de la première position à la cinquième position en 2.5 secondes, et vice-versa. La combinaison de ces trois éléments forme un organe de démultiplication qui permet d'obtenir de telles performances avec un moteur standard.

Un système de commande du dispositif conforme à l'invention pourra comprendre un module de programmation et l'électronique associée afin de permettre au passager de contrôler le pulseur et le moteur du mécanisme d'actionnement électrique. Préférentiellement, le passager peut contrôler le distributeur de fragrances à partir d'une commande au volant.

Le système de commande permet au conducteur de mettre le distributeur de fragrances sous tension, de choisir l'intensité de la fragrance diffusée, de choisir le débit du flux d'air diffusé et/ou de choisir la fragrance. Avantageusement, le système de commande indique lorsqu'une fragrance de la cartouche est vide.

Dans un premier temps, le système de commande permet au passager de choisir la fragrance qu'il souhaite diffuser. Ensuite, il peut également régler le débit du flux d'air généré par le pulseur. Le système de commande permet également au passager de régler l'intensité de la fragrance qu'il souhaite diffuser.

Dans un mode de réalisation de la présente invention, le système de commande permet au passager de préprogrammer une séquence de diffusion qu'il souhaite en choisissant une durée de diffusion, une fragrance et son intensité. L'intensité pourra également varier au cours du temps ; il est dès lors possible de commencer par une diffusion totale qui devient partielle au cours du temps. Le système de commande permet également de choisir une fréquence de diffusion. Par exemple, le passager peut choisir de diffuser une certaine fragrance pendant une durée choisie, et puis ne rien diffuser pendant une autre durée.

## Revendications

1. Cartouche (130) comprenant un ou une pluralité d'agents volatils, notamment de fragrances, ledit agent ou chacun desdits agents étant logés dans une cavité (340) destinée à être balayée par un flux d'air (120), ladite cartouche étant configurée pour équiper un dispositif de diffusion, en particulier pour véhicules automobiles.

2. Cartouche selon la revendication précédente, dans laquelle au moins une desdites cavités (340) est vide.

3. Cartouche selon la revendication précédente, dans laquelle des cavités occupées par une fragrance alternent avec des cavités vides.

4. Cartouche (130) selon l'une des revendications précédentes, dans laquelle les cavités sont configurées pour que le flux d'air traverse la cartouche de part en part en passant par l'une ou plusieurs des cavités de la cartouche.

5. Cartouche (130) selon la revendication précédente, la cartouche comprenant un corps (360) et un couvercle (350) dont la forme est parallélépipédique et une base est sensiblement carrée, le corps étant compartimenté pour définir les cavités, un fond dudit corps comprenant des entrées du flux d'air desdites cavités, et le couvercle comprenant des sorties du flux d'air desdites cavités.

6. Cartouche selon l'une quelconque des revendications précédentes, dans lequel les différentes cavités (340) de la cartouche (130) forment des portions angulaires d'un anneau.

7. Cartouche selon la revendication précédente dans laquelle l'angle balayé par des ouvertures de chaque portion angulaire est inférieur à 60 degrés.

8. Cartouche selon la revendication précédente dans laquelle les ouvertures sont revêtues d'un grillage ou d'un film poreux.

9. Cartouche selon l'une des revendications précédentes, ladite cartouche (130) étant configurée pour être amovible par rapport audit dispositif.

10. Cartouche selon la revendication précédente, dans lequel la cartouche (130) comprend une façade d'introduction (310) dans un boîtier (100) du dispositif.

11. Cartouche selon l'une des revendications 7 ou 8, la cartouche étant pourvue d'une nervure longitudinale (315) de guidage située le long de trois faces verticales configurées pour s'insérer dans le boîtier (100).

12. Cartouche selon l'une quelconque des revendications précédentes, comprenant une fente (320) permettant le passage de la cartouche (130) en présence d'un sélecteur (125a, 125b) du dispositif, en particulier le passage d'un axe (135) d'entraînement dudit sélecteur, lorsque la cartouche est insérée dans un boîtier (100) du dispositif.

13. Cartouche selon la revendication précédente, dans laquelle l'axe mobile (135) se trouve, dans une position d'utilisation, au centre de la cartouche dans le fond de la fente.

14. Cartouche selon l'une quelconque des revendications précédentes dans lequel le ou lesdits agents volatils comprennent des billes odorantes.

15. Ensemble d'un dispositif de diffusion et d'une cartouche selon l'une quelconques de revendications 1 à 14.
